# EUROPEAN PATENT APPLICATION

(11) **EP 4 755 408 A1**
(43) Date of publication of application: **10.06.2026**
(21) Application number: 24871697.9
(22) Date of filing: 29.08.2024
(51) Int. Cl.: A61L 31/16, A61F 2/82, A61L 31/14

(54) **LIVING BODY INDWELLING OBJECT**

(30) Priority: 29.09.2023 JP 2023169879
(71) Applicant: Terumo Kabushiki Kaisha, Tokyo 151-0072 (JP)
(72) Inventor: MATSUSHITA, Shuhei, Ashigarakami-gun, Kanagawa 259-0151 (JP); TANI, Kazuyoshi, Ashigarakami-gun, Kanagawa 259-0151 (JP); MORI, Yuhei, Ashigarakami-gun, Kanagawa 259-0151 (JP)
(74) Representative: Casalonga
(86) International application number: PCT/JP2024/030858
(87) International publication number: WO 2025/069896

(57) **Abstract**

[Object] Provided is a living body indwelling object capable of effectively suppressing the onset of inflammation derived from a porous structure made of a biodegradable polymer while preventing scattering of a plaque and a thrombus at the time of stent expansion.

[Solving Means] A living body indwelling object 100 includes an expandable tubular stent 10 and a porous structure 20 made of a first biodegradable polymer, the porous structure being disposed to cover the stent, in which the stent includes a drug carrying portion 18 containing a second biodegradable polymer and a drug, and a decomposition time of the porous structure is substantially the same as a decomposition time of the drug carrying portion or shorter than a decomposition time of the drug carrying portion.

## Description

### Technical Field

The present invention relates to a living body indwelling object.

### Background Art

A stent is a medical tool that is indwelt after being delivered to a lesion in a living body lumen by a stent delivery system, expands the lesion such as a stenosed site or an occluded site, and secures the lumen in order to treat various diseases caused by stenosis or occlusion of the living body lumen such as a blood vessel. A general stent has a linear strut that forms a cylindrical outer periphery with gaps formed. When the stent is delivered to the lesion and then expanded (expanded in diameter) in the living body lumen, the stent imparts tensile strength (expansion force) to the lesion. The stent maintains the lesion in a spread state by maintaining the tensile strength over a predetermined period, and secures the lumen of the living body lumen.

For example, Patent Literature 1 discloses a mesh-covered stent in which a mesh is arranged so as to cover the outer periphery of the stent in order to prevent peripheral emboli at the time of stent implantation. In the mesh-covered stent, the mesh having an expandable knitted stitch structure expands following expansion of the stent, thereby preventing scattering of a plaque and a thrombus when the stent expands.

### Citation List

### Patent Literature

Patent Literature 1: WO 2006/126182 A2

### Summary of Invention

### Technical Problem

In the mesh-covered stent, a polymer mesh may be adopted from the viewpoints of improving the followability of the mesh when the stent is expanded and the delivery property to the lesion, reducing the influence that can be given to the living body lumen after treatment, and the like. However, when a polymer mesh is adopted, the following points are concerned.

In general, polymer materials are more likely to cause tissue inflammation than metal materials. From such a background, in a known drug-eluting stent, when a polymer is used as a drug carrier, a biodegradable polymer or a bioinert polymer is often selected. However, it is said that polymer-derived inflammation can be suppressed by a drug released (eluted) from the stent. Therefore, when a biodegradable polymer is used as a constituent material of a stent, it is considered that the influence of polymer-derived inflammation can be reduced by using a polymer that rapidly disappears after the release of the drug is completed.

Based on the above findings, the inventors of the present invention have found that in a mesh-covered stent (living body indwelling object) including a polymer mesh (porous structure), by adjusting the time from the time of indwelling of the mesh-covered stent until the polymer mesh is decomposed and disappeared and the time from the time of indwelling of the mesh-covered stent until the release of the drug is completed, it is possible to effectively suppress the onset of inflammation derived from the polymer mesh while preventing the scattering of a plaque and a thrombus at the time of stent expansion.

An object of the present invention is to provide a living body indwelling object capable of effectively suppressing the onset of inflammation derived from a porous structure made of a biodegradable polymer while preventing scattering of a plaque and a thrombus at the time of stent expansion.

### Solution to Problem

The above object of the present invention is achieved by any one of the following means (1) to (15).
(1) A living body indwelling object including an expandable tubular stent and a porous structure made of a first biodegradable polymer, the porous structure being disposed to cover the stent, wherein
   the stent includes a drug carrying portion containing a second biodegradable polymer and a drug, and
   a decomposition time of the porous structure is substantially the same as a decomposition time of the drug carrying portion or shorter than a decomposition time of the drug carrying portion.
(2) The living body indwelling object according to (1), wherein the stent is made of a non-biodegradable material, and
   the drug carrying portion is disposed only in a portion of a surface of a ring forming the stent, the portion facing the porous structure.
(3) The living body indwelling object according to (1), wherein the stent is made of a non-biodegradable material, and
   the drug carrying portion is disposed to cover an entire range of a surface of a ring forming the stent.
(4) The living body indwelling object according to (1), wherein the entire stent is configured by the drug carrying portion.
(5) The living body indwelling object according to any one of (1) to (4), wherein the first biodegradable polymer and the second biodegradable polymer are the same polymer, and the first biodegradable polymer and the second biodegradable polymer are selected from the group consisting of a homopolymer of one monomer selected from the group consisting of lactic acid, caprolactone, glycolic acid, dioxanone, butyrolactone, valerolactone, hydroxybutyric acid, and trimethylene carbonate, or a copolymer of two or more monomers.
(6) The living body indwelling object according to any one of (1) to (4), wherein the first biodegradable polymer and the second biodegradable polymer are different polymers, the first biodegradable polymer is at least one selected from the group consisting of polyglycolic acid, polycaprolactone, a glycolic acid-lactic acid copolymer, an ε-caprolactone-lactic acid copolymer, and a glycolic acid-ε-caprolactone copolymer, and the second biodegradable polymer is at least one selected from the group consisting of polylactic acid, a lactic acid-ε-caprolactone copolymer, a lactic acid-glycolic acid copolymer, and polydioxanone.
(7) The living body indwelling object according to (6), wherein a molecular weight of the first biodegradable polymer is 120,000 or more and 180,000 or less, and a molecular weight of the second biodegradable polymer is 100,000 or more and 200,000 or less.
(8) The living body indwelling object according to (6) or (7), wherein the first biodegradable polymer is at least one selected from the group consisting of polyglycolic acid and a polyglycolic acid-polylactic acid copolymer, and the second biodegradable polymer is at least one selected from the group consisting of polylactic acid, a lactic acid-ε-caprolactone copolymer, and polydioxanone.
(9) The living body indwelling object according to any one of (6) to (8), wherein a molecular weight of the first biodegradable polymer is 140,000 or more and 180,000 or less, and a molecular weight of the second biodegradable polymer is 100,000 or more and 120,000 or less.
(10) The living body indwelling object according to any one of (1) to (9), wherein a decomposition time of the porous structure is within 4 months.
(11) The living body indwelling object according to any one of (1) to (10), wherein a decomposition time of the drug carrying portion is more than 4 months.
(12) The living body indwelling object according to any one of (1) to (11), wherein a number average molecular weight in a state where the porous structure and the drug carrying portion are decomposed is 50 to less than 800.
(13) The living body indwelling object according to (2) or (3), wherein the non-biodegradable material is a metal material or a polymer material.
(14) The living body indwelling object according to any one of (1) to (13), wherein the drug contains an anti-inflammatory agent.
(15) The living body indwelling object according to any one of (1) to (14), wherein a ratio of the drug in the drug carrying portion is 40 mass% or more and 55 mass% or less with respect to a total amount of the second biodegradable polymer and the drug.

### Advantageous Effects of Invention

According to the above-described living body indwelling object, after a certain period of time elapses after indwelling, the porous structure is decomposed and disappeared, and the release of the drug from the drug carrying portion disposed in the stent is continued until the porous structure is decomposed and disappeared. Therefore, the above-described living body indwelling object can effectively suppress the onset of inflammation derived from the porous structure made of a biodegradable polymer while preventing scattering of a plaque and a thrombus at the time of stent expansion.

### Brief Description of Drawings

[Fig. 1] Fig. 1 is a schematic plan view illustrating a stent delivery system including a living body indwelling object according to an embodiment.
[Fig. 2] Fig. 2 is a schematic plan view illustrating a reduced diameter state of the living body indwelling object according to the embodiment.
[Fig. 3] Fig. 3 is a schematic plan view illustrating an expanded state (increased diameter state) of the living body indwelling object according to the embodiment.
[Fig. 4] Fig. 4 is a partially enlarged view illustrating a part of the living body indwelling object in the state illustrated in Fig. 3 in an enlarged manner.
[Fig. 5] Fig. 5 is an enlarged cross-sectional view illustrating a cross section of a part of a stent and a porous structure in an enlarged manner, and is a view illustrating an arrangement example of a drug carrying portion.
[Fig. 6] Fig. 6 is an enlarged cross-sectional view illustrating a cross section of a part of the stent and the porous structure in an enlarged manner, and is a view illustrating an arrangement example of the drug carrying portion.
[Fig. 7] Fig. 7 is an enlarged cross-sectional view illustrating a cross section of a part of the stent and the porous structure in an enlarged manner, and is a view illustrating an arrangement example of the drug carrying portion.

### Description of Embodiments

An embodiment of the present invention will be described below with reference to the accompanying drawings. Note that the following description does not limit the technical scope or the significance of each term disclosed in the claims. The dimensional ratios in the drawings are exaggerated for convenience of description and may be different from actual ratios.

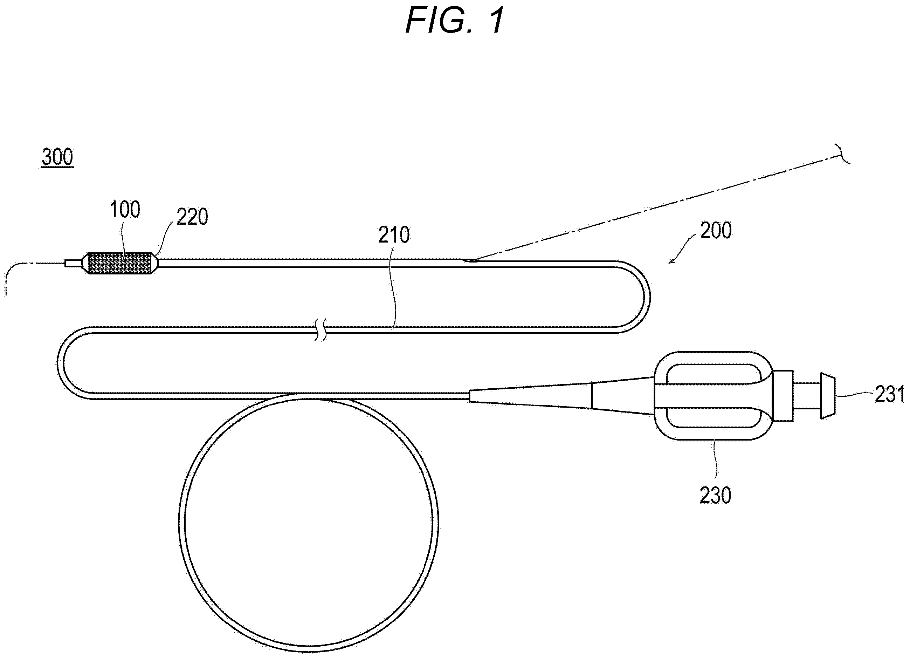

Hereinafter, a living body indwelling object 100 according to the present embodiment will be described with reference to Figs. 1 to 7.

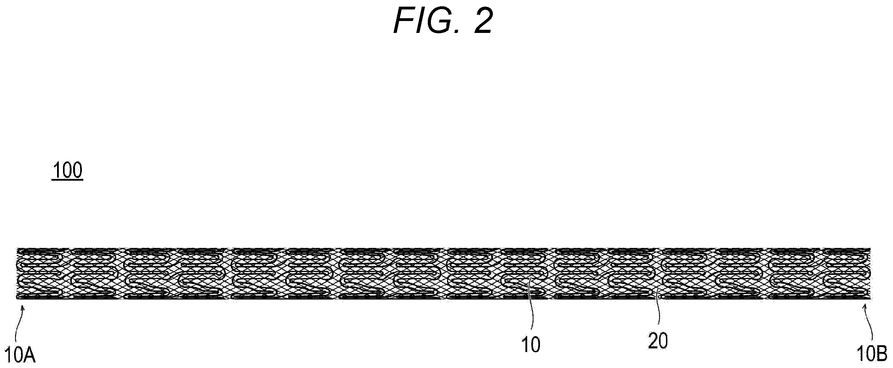

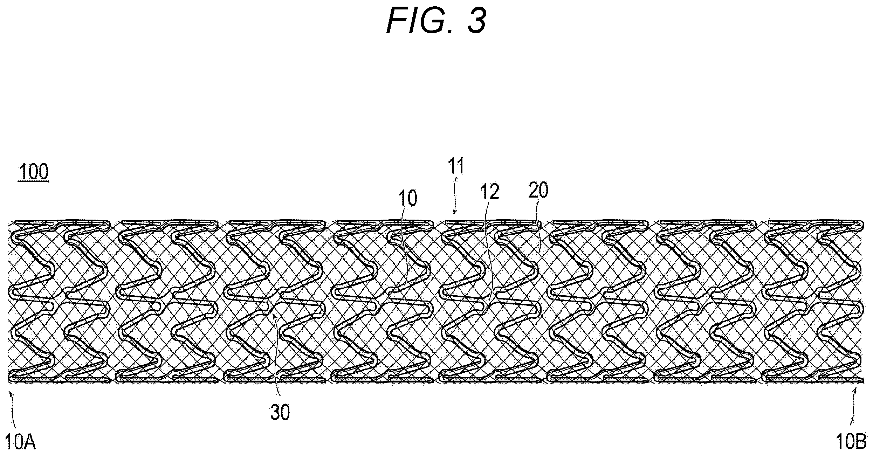

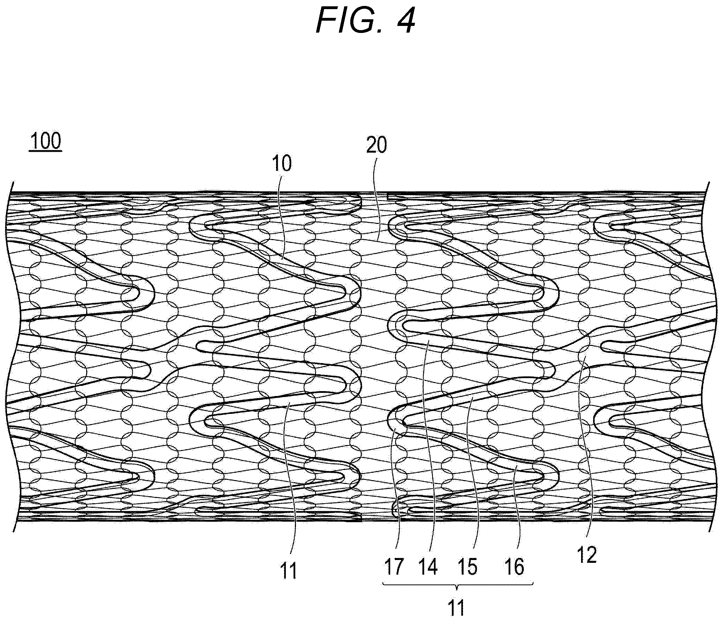

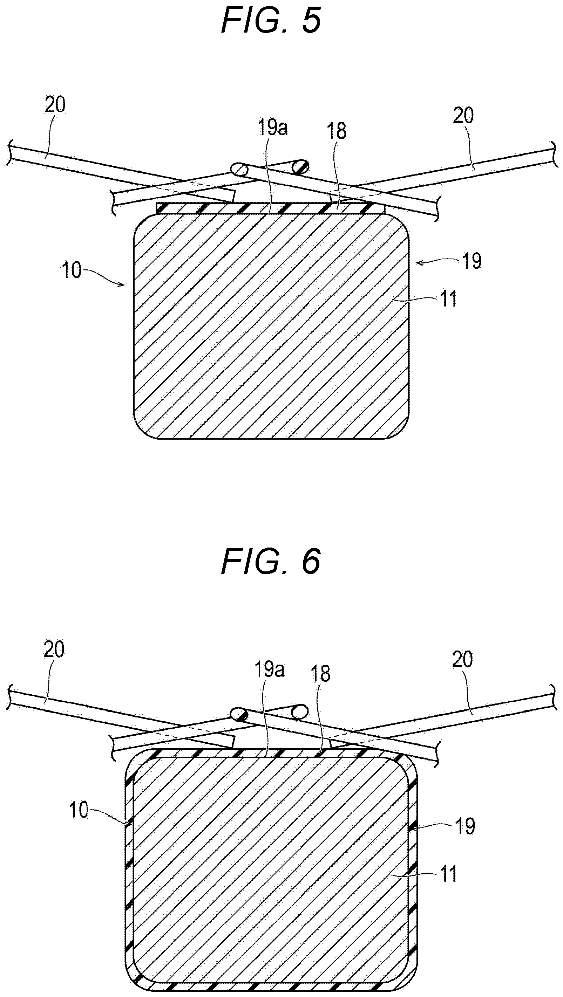

Fig. 1 is a schematic plan view illustrating a stent delivery system 300 including the living body indwelling object 100 according to an embodiment of the present invention. Fig. 2 is a schematic plan view illustrating a reduced diameter state of the living body indwelling object 100 according to the embodiment. Fig. 3 is a schematic plan view illustrating an increased diameter state of the living body indwelling object 100 according to the embodiment. Fig. 4 is a partially enlarged view illustrating the living body indwelling object 100 according to the embodiment. Figs. 5 to 7 are enlarged cross-sectional views schematically illustrating arrangement examples of a drug carrying portion 18 in a stent 10.

In the present specification, a direction in which the living body indwelling object 100 extends is referred to as "axial direction". The axial direction is a direction from a distal end 10A to a proximal end 10B illustrated in Fig. 2. Note that a side to be inserted into a living body is referred to as a "distal end side", and a side which is opposite to the distal end side and on which a surgeon operates a medical device is referred to as a "proximal end side". A direction orthogonal to the "axial direction" is referred to as "radial direction" of the living body indwelling object 100.

As illustrated in Fig. 1, the stent delivery system 300 includes the living body indwelling object 100 and a balloon catheter 200.

### <Balloon Catheter 200>

The balloon catheter 200 is used to deliver the living body indwelling object 100 in a contracted state to a lesion, and to expand and indwell the living body indwelling object 100 near the lesion.

The balloon catheter 200 includes an elongated catheter body 210, a balloon 220 provided at a distal end of the catheter body 210, and a hub 230 fixed to a proximal end of the catheter body 210.

The catheter body 210 includes an outer tube and an inner tube disposed inside the outer tube.

An expansion lumen through which an expansion fluid for expanding the balloon 220 flows is formed inside the outer tube. A distal end portion of the outer tube is fixed to a proximal end portion of the balloon 220. A proximal end portion of the outer tube is fixed to the hub 230.

A guide wire lumen into which a guide wire is inserted is formed inside the inner tube. A distal end portion of the inner tube penetrates the inside of the balloon 220 and is opened on the distal end side of the balloon 220. A proximal end portion of the inner tube penetrates a side wall of the outer tube on the proximal end side of the balloon 220 and is fixed to the outer tube.

A constituent material of the catheter body 210 is preferably a material having a certain degree of flexibility, and examples thereof include polyolefins such as polyethylene, polypropylene, polybutene, an ethylenepropylene copolymer, an ethylene-vinyl acetate copolymer, and an ionomer, or a mixture of two or more kinds thereof, thermoplastic resins such as a polyvinyl chloride resin, polyamide, a polyamide elastomer, polyester, a polyester elastomer, polyurethane, and a fluororesin, a silicone rubber, and a latex rubber.

The balloon 220 is a member that pushes and expands a stenosed site by expanding inside the stenosed site (lesion), for example. The distal end side of the balloon 220 is fixed to an outer wall surface of the inner tube. The proximal end side of the balloon 220 is fixed to an outer wall surface of the distal end portion of the outer tube. Therefore, the inside of the balloon 220 communicates with the expansion lumen formed in the outer tube. The balloon 220 allows inflow of the expansion fluid from a proximal opening 231 through the expansion lumen. The balloon 220 is expanded due to the inflow of the expansion fluid, and is contracted and folded by discharging the expansion fluid that has flowed therein.

A constituent material of the balloon 220 is preferably a flexible material that expands and contracts when the expansion fluid flows in and out, and examples thereof include polymer materials such as a polyolefin, a crosslinked polyolefin, polyester, a polyester elastomer, polyvinyl chloride, polyurethane, a polyurethane elastomer, polyphenylene sulfide, polyamide, a polyamide elastomer, and a fluororesin, a silicone rubber, and a latex rubber. The constituent material of the balloon 220 is not limited to a mode in which the above-described polymer material is used alone, and a film in which the above-described polymer materials are appropriately laminated may be applied. The expansion fluid may be a gas or a liquid, and examples thereof include gases such as a helium gas, a CO2 gas and an O2 gas, and liquids such as physiological saline and an X-ray contrast agent.

The hub 230 includes the proximal opening 231 communicating with the expansion lumen of the outer tube. The proximal opening 231 functions as a port through which the expansion fluid flows in and out.

A constituent material of the hub 230 is not particularly limited, and examples thereof include thermoplastic resins such as polyethylene, polyurethane, polyester, polypropylene, polycarbonate, polyamide, polysulfone, polyarylate, and a methacrylate-butylene-styrene copolymer.

### <Living Body Indwelling Object 100>

The living body indwelling object 100 according to the present embodiment is used, for example, to treat a stenosed site or an occluded site generated in a blood vessel, a bile duct, a trachea, an esophagus, a urethra, or other living body lumens. The living body indwelling object 100 is a so-called balloon-expandable type that is attached in a state of being crimped to the balloon 220, delivered to a lesion, and then expanded and indwelt in the lesion. Note that the living body indwelling object 100 can also be formed of a so-called self-expandable stent.

As illustrated in Figs. 2 to 7, in general, the living body indwelling object 100 generally includes an expandable tubular stent 10 and a porous structure 20 made of a first biodegradable polymer disposed so as to cover the stent 10. The stent 10 includes the drug carrying portion 18 containing a second biodegradable polymer and a drug.

The stent 10 has a substantially cylindrical shape in which a lumen extending in the axial direction is formed. The distal end 10A is located at the end portion on the distal end side of the stent 10, and the proximal end 10B is located at the end portion on the proximal end side of the stent 10.

As illustrated in Figs. 2 and 3, the stent 10 is formed so as to be capable of expanding (state illustrated in Fig. 3) and contracting (state illustrated in Fig. 2) in the radial direction. As illustrated in Figs. 3 and 4, the stent 10 includes linear rings 11 that form a cylindrical outer periphery having gaps, and link portions 12 that connect the rings 11 to each other through the gaps.

As illustrated in Fig. 4, the wavy ring 11 includes a plurality of first strut portions 15 formed of a straight line or a curved line, a plurality of second strut portions 16 formed of a straight line or a curved line, and a plurality of curved portions 17 formed between the first strut portions 15 and the second strut portions 16. As illustrated in Fig. 4, the ring 11 includes a plurality of third strut portions 14 which are adjacent to one side (for example, the left side in Fig. 4) of the link portion 12 in the axial direction, are provided in a pair in a circumferential direction (up and down in Fig. 4), and are formed of a straight line or a curved line.

The rings 11 are sequentially disposed along the axial direction, and the rings 11 adjacent to each other in the axial direction are integrated by the link portion 12. Therefore, by increasing or decreasing the number of rings 11, the stent 10 having a desired length can be easily obtained.

As illustrated in Figs. 3, 4, and 5, the porous structure 20 having a large number of penetrating voids is disposed so as to cover the outer periphery of the stent 10 (a portion facing the outside of a surface 19 of the ring 11). As illustrated in Figs. 2 and 3, the porous structure 20 is configured to be expandable following expansion of the stent 10. By disposing the porous structure 20 on the outer periphery of the stent 10, when the stent 10 is expanded in the blood vessel, the porous structure 20 is urged against the blood vessel by the stent 10, so that the breakage of the porous structure 20 due to the pressure of the blood flow flowing in the blood vessel is reduced.

The porous structure 20 extends along the axial direction of the stent 10 and has a knitted stitch structure. Gaps of the knitted stitch structure of the porous structure 20 can be configured to be smaller than the gaps between the adjacent rings 11. By making the network of the porous structure 20 smaller than the gaps of the rings 11 in this manner, it is possible to prevent a plaque and a thrombus from scattering when the stent 10 is expanded at the lesion. As a result, it is possible to prevent peripheral emboli from being generated by a plaque or a thrombus.

The porous structure 20 includes, for example, any of a knitted product (knit), a textile product (braid), and a molded product. When the porous structure 20 is a knitted product, the porous structure 20 can be configured by, for example, jersey knitting. By configuring the porous structure 20 by jersey knitting, when the stent 10 is expanded, it is possible to suppress shortening of the length of the porous structure 20 in the axial direction along with expansion of the stent 10. Note that, since the jersey knitting is a known knitting method, the detailed description in the present specification will be omitted. When the porous structure 20 is a textile product, the textile product is formed by a known weaving method. When the porous structure 20 is a molded product, the porous structure 20 is formed of a cylindrical sheet having regularly arranged voids. The size, shape, and number of knit stitches and weave stitches of the porous structure 20 are not particularly limited as long as peripheral emboli at the time of expansion of the stent 10 can be prevented.

The yarn diameter of the porous structure 20 is not particularly limited, but can be, for example, 20 µm.

Note that the porous structure 20 is not limited to one formed by jersey knitting. The porous structure 20 can also be made of, for example, a knitted product, a textile product (braid), or the like. The size, shape, number, and the like of knit stitches of the porous structure 20 are not particularly limited as long as peripheral emboli due to a plaque or a thrombus can be prevented from occurring when the stent 10 is expanded. The porous structure 20 can be fixed at an arbitrary position such as the distal end 10A, the proximal end 10B, and the link portions 12 of the stent 10. A method for fixing the porous structure 20 to the stent 10 is not particularly limited, and for example, any method such as adhesion fixation with a polymer or paraffin, and fixation using a mechanical means such as a binding band, a clip, or a thread can be used.

Hereinafter, preferred constituent materials and operational effects of the living body indwelling object 100, preferred arrangement examples of the drug carrying portion 18, and the like will be described.

For example, as in a first embodiment illustrated in Fig. 5, the drug carrying portion 18 can be disposed only in a portion 19a facing at least the porous structure 20 (hereinafter, referred to as "facing portion 19a") in cross-sectional view orthogonal to the axial direction, of the surface 19 of the ring 11 forming the stent 10. However, as described later, the drug carrying portion 18 may be in any form as long as it is present on at least a part of the surface 19 of the ring 11 forming the stent 10 or at least a part of the stent 10 itself. The drug carrying portion 18 may exist in at least a part or all of the plurality of first strut portions 15, second strut portions 16, and third strut portions 14 of the ring 11, and may not exist or may exist in the plurality of link portions 12 and/or curved portions 17. Preferably, the drug carrying portion 18 exists in the plurality of first strut portions 15, second strut portions 16, and third strut portions 14, and does not exist in the link portions 12 and the curved portions 17. As a result, it is possible to reduce the phenomenon that the drug carrying portion 18 is peeled off at the time of expansion.

In the first embodiment, the drug carrying portion 18 is formed only on the facing portion 19a of the ring 11. According to the first embodiment, since the drug carrying portion 18 is selectively disposed in the facing portion 19a, the drug contained in the drug carrying portion 18 is selectively released only from the side where the porous structure 20 is disposed (facing portion 19a side). Therefore, according to the first embodiment, it is possible to more effectively suppress and prevent the inflammatory reaction induced by the porous structure 20 with a drug amount smaller than that when the drug carrying portion 18 is disposed to cover the entire range of the surface 19 of the ring 11. That is, the risk of inflammation due to the contact between the porous structure 20 and a biological tissue in the vicinity of the facing portion 19a can be effectively reduced and eliminated.

In the first embodiment, the shape and size (cross-sectional shape, range provided in the facing portion 19a, and the like) of the drug carrying portion 18 are not particularly limited, and can be appropriately set according to the cross-sectional shape and size of the ring 11. For example, when the cross-sectional shape of the ring 11 is square, substantially square, rectangular, or substantially rectangular, the drug carrying portion 18 can be formed in the entire range of the surface of the ring 11 that can be in contact with the porous structure 20. The thickness of the drug carrying portion 18 is not particularly limited, and is appropriately selected according to a desired decomposition time, the content of the drug, and the like. The thickness of the drug carrying portion 18 may be, for example 1 to 20 µm.

In a second embodiment of the present invention, as illustrated in Fig. 6, the stent 10 is made of a non-biodegradable material, and the drug carrying portion 18 can be disposed to cover the entire range in cross-sectional view orthogonal to the axial direction of the surface 19 of the ring 11 forming the stent 10. According to the second embodiment, the drug carrying portion 18 is disposed in a region other than a contact region with the porous structure 20. Therefore, according to the second embodiment, since the drug concentration can be sufficiently maintained as compared with a case where the drug carrying portion 18 is disposed only on the facing portion 19a side of the ring 11, the inflammatory reaction induced by the porous structure 20 can be sufficiently suppressed and prevented. In the living body indwelling object 100, for example, an anti-inflammatory agent can be disposed on the side where the porous structure 20 and a biological tissue are in contact with each other, and another drug that can be used for treatment of a subject can be disposed on the other covering portion. Therefore, according to the second embodiment, it is possible to more effectively suppress and prevent the inflammatory reaction induced from the porous structure 20, and it is also possible to simultaneously perform other required treatments.

In the second embodiment, the thickness of the drug carrying portion 18 is not particularly limited, and is appropriately selected according to the decomposition time, the content of the drug, and the like. The thickness of the drug carrying portion 18 may be, for example 0.5 to 10 µm.

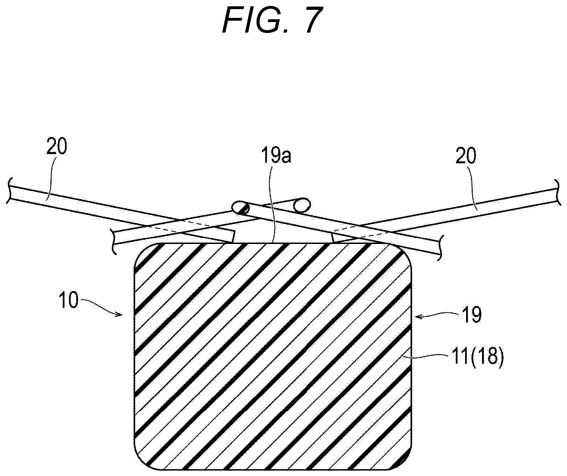

In a third embodiment of the present invention, as illustrated in Fig. 7, the entire stent 10 (the entire ring 11 forming the stent 10) is configured by the drug carrying portion 18. That is, the stent 10 itself may function as the drug carrying portion 18. According to the third embodiment, since it is not necessary to separately form the drug carrying portion 18 in the stent 10, the number of manufacturing steps can be reduced, which is preferable from the viewpoint of mass production. As in the second embodiment, for example, it is possible to dispose a large amount of anti-inflammatory agent on the contact region side (facing portion 19a side) in contact with the porous structure 20 in the stent 10, and to dispose another drug that can be used for treatment of a subject in the other portion. Therefore, according to the third embodiment, it is possible to more effectively suppress and prevent the inflammatory reaction induced from the porous structure 20, and it is also possible to simultaneously perform other required treatments.

Note that, among the first to third embodiments, the first embodiment and the second embodiment are preferable, and the first embodiment is more preferable from the viewpoint of the effect of further suppressing and preventing an inflammatory reaction, a higher tensile strength of the stent 10, a longer tensile strength maintaining period, and the like.

In the first embodiment and the second embodiment, as a non-biodegradable material that can be used for the stent 10, carbon fiber, a metal material, a polymer material, and the like can be used. Preferably, the non-biodegradable material is a metal material or a polymer material. From the viewpoint of an effect of further reducing inflammation and the like, the non-biodegradable material is particularly preferably a metal material. Here, a metal material used when the stent 10 is made of a metal material is not particularly limited, and a metal material usually used in the product field of the stent can be used. Specific examples thereof include stainless steels such as SUS304, SUS316, SUS316L, SUS420J2, and SUS630, tantalum, titanium, a nickel-titanium alloy, a tantalum-titanium alloy, a nickel-aluminum alloy, Inconel, gold, platinum, iridium, tungsten, and cobalt-based alloys such as a cobalt-chromium (Co-Cr) alloy. Among stainless steels, SUS316L having the best corrosion resistance is preferable. Among the cobalt-based alloys, MP35N, L605, and the like are preferable. A polymer material that is used when the stent 10 is made of a polymer material is not particularly limited, and a polymer material usually used in the product field of the stent 10 can be used. Specific examples thereof include polyolefins such as polyethylene and polypropylene, aromatic polyesters such as polyethylene terephthalate, cellulose-based polymers such as cellulose acetate and cellulose nitrate, and fluorine-containing polymers such as polytetrafluoroethylene and a tetrafluoroethylene-ethylene copolymer.

The stent 10 can be suitably formed of a material appropriately selected from the materials exemplified above according to the application place and the like. For example, when the stent 10 is formed of a metal material, since the metal material is excellent in strength, it is possible to more reliably indwell the living body indwelling object 100 in the lesion. When the stent 10 is formed of a polymer material, since the polymer material is excellent in flexibility, an effect that is excellent in reachability (delivery property) of the living body indwelling object 100 to the lesion is exhibited.

When the stent 10 is a self-expandable type, a superelastic alloy such as a nickel-titanium alloy or the like is preferable since a restoring force to an original shape is required. Meanwhile, when the stent 10 is a balloon-expandable type, stainless steel or the like is preferable in that shape restoration after expansion hardly occurs. When the stent 10 is made of carbon fiber, an excellent effect is exhibited in terms of high strength, excellent flexibility, and high safety in vivo.

The material, shape, size, and the like of the stent 10 are not particularly limited as long as it can be indwelt in a lesion generated in a lumen in a living body such as a blood vessel, a bile duct, a trachea, an esophagus, or a urethra. For example, when the stent is used in the coronary artery of the heart, the outer diameter of the stent 10 before expansion is usually, for example, 1.0 to 3.0 mm. The thickness (thickness on the cross-sectional view illustrated in Figs. 5 to 7) of the stent 10 is, for example, 0.05 to 0.25 mm. The length (length in the axial direction) of the stent 10 is, for example, 5 to 50 mm.

A method for manufacturing the stent 10 is not particularly limited, and may be appropriately selected from commonly used manufacturing methods according to the structure and material of the stent 10. For example, manufacturing methods using an etching technique such as laser etching or chemical etching, and laser cut technique can be selected.

In the living body indwelling object 100 of the present invention, the decomposition time of the porous structure 20 is substantially the same as the decomposition time of the drug carrying portion 18 or shorter than the decomposition time of the drug carrying portion 18. By adopting such a configuration, the following effects can be exhibited.

For the purpose of suppressing and preventing peripheral emboli associated with scattering of a plaque and a thrombus at the time of indwelling of the stent, covering a metal stent with a porous structure has been studied (for example, WO 2008/062414 A). However, the polymer material generally has a problem of easily inducing inflammation in the contacted tissue as compared with the metal material. On the other hand, in the present invention, the porous structure 20 is made of a biodegradable polymer, and the decomposition time of the porous structure 20 is equal to or less than the decomposition time of the drug carrying portion 18. That is, the drug is continuously released from the drug carrying portion 18 until the porous structure 20 is decomposed and disappeared, and the inflammation of the biological tissue (for example, tissue located on an inner wall surface of a blood vessel) in contact with the porous structure 20 can be suppressed and prevented by the drug released from the drug carrying portion 18. Therefore, according to the present invention, it is possible to achieve both suppression and prevention of peripheral emboli and reduction and elimination of the risk of tissue inflammation due to contact with the porous structure 20.

The decomposition time of the porous structure 20 and the decomposition time of the drug carrying portion 18 can be controlled by the type of the first biodegradable polymer constituting the porous structure 20 and the type of the second biodegradable polymer constituting the drug carrying portion 18. Since the sizes of the first biodegradable polymer and the second biodegradable polymer that constitutes the drug carrying portion 18 have little or no influence on the decomposition time (decomposition rate, solubility) (in a material in which the entire material is uniformly decomposed), the decomposition time can be suitably controlled by the first biodegradable polymer constituting the porous structure 20 and the second biodegradable polymer constituting the drug carrying portion 18. That is, the decomposition time of the first biodegradable polymer is substantially the same as the decomposition time of the second biodegradable polymer or shorter than the decomposition time of the second biodegradable polymer.

In the present specification, the "decomposition time" of the porous structure 20, the drug carrying portion 18, the first biodegradable polymer, and the second biodegradable polymer is, for example, a value measured according to the following method.

### [Method for Measuring Decomposition Time]

0.3 mg of each of the first biodegradable polymer and the second biodegradable polymer (these are collectively referred to as "sample") are weighed in a screwed test tube. Next, phosphate buffered saline (PBS) at pH 7.4 is injected into the 15 ml line of the test tube. At this time, phosphate buffered saline (PBS) is used as a simulated body fluid. Thereafter, the test tube is stored in a thermostatic bath at 37°C for a predetermined period (0, 1, 3, 7, 14 and 30 days) to perform hydrolysis. After a lapse of a predetermined period of time, the screwed test tube is taken out from the thermostatic bath, PBS is sucked from the screwed test tube with a Pasteur pipette, and then moisture in the test tube is completely evaporated by drying under reduced pressure. Thereafter, a mobile phase solvent (5 mM sodium trifluoroacetate containing hexafluoroisopropanol) is added in the test tube so as to have a sample concentration of 1 mg/ml, the sample is completely dissolved, and then the weight average molecular weight (Mw) and the number average molecular weight (Mn) are measured according to the following method.

### (Method for Measuring Weight Average Molecular Weight (Mw) and Number Average Molecular Weight (Mn))

The weight average molecular weight (Mw) and the number average molecular weight (Mn) are values measured under the following measurement conditions by gel permeation chromatography (GPC) using polymethyl methacrylate as a standard substance:

### (Measurement Conditions)

Apparatus: Semi-micro GPC system LC-VP (manufactured by Shimadzu Corporation)
Detector: Shodex (registered trademark) RI-104 (manufactured by Showa Denko K.K.)
Column: Shodex (registered trademark) GPC LF-404 (manufactured by Showa Denko K.K.)
Column temperature: 40°C
Mobile phase solvent: 5 mM sodium trifluoroacetatecontaining hexafluoroisopropanol (HFIP)
Sample concentration: 1 mg/ml
Flow rate: 0.30 mL/min
Injection volume: 10 µL
Sample preparation: 1 mL of a mobile phase solvent is added to 1 mg of a sample to be measured to dissolve the sample, and then the solution is filtered through a 0.45 µm PTFE membrane filter.

Next, a logarithmic plot of the obtained number average molecular weight (Mn) was created, the time at which Mn became 50 to 800 (preferably 50 to 150, more preferably 100) was specified from a linear regression equation of this plot, and this time was defined as a decomposition disappearance period of the mesh.

Note that, in the living body indwelling object 100, the period during which the first biodegradable polymer constituting the porous structure 20 and the second biodegradable polymer contained in the drug carrying portion 18 are decomposed and disappeared, that is, the number average molecular weight becomes 50 to 800 (preferably 50 to 150, more preferably 100) is preferably 1.9 months or more and 4.0 months or less.

The first biodegradable polymer and the second biodegradable polymer are not particularly limited, and for example, known biodegradable polymers such as those described in JP 2011-528275 A, JP 2008-514719 A, WO 2008/1952 A, JP 2004-509205 A, and the like can be used. Specifically, (1) a polymer selected from the group consisting of aliphatic polyester, polyester, polyacid anhydride, polyorthoester, polycarbonate, polyphosphazene, polyphosphoric acid ester, polyvinyl alcohol, polypeptide, polysaccharide, protein, and cellulose; (2) a copolymer composed of two or more monomers constituting the (1) above can be used. Here, the aliphatic polyester is not particularly limited, and examples thereof include polylactic acid (PLA) such as poly-L-lactic acid (PLLA), poly-D-lactic acid (PDLA), and poly-DL-lactic acid (PDLLA), polyglycolic acid (PGA), polyhydroxybutyric acid, polyhydroxyvaleric acid, polyhydroxypentanoic acid, polyhydroxyhexanoic acid, polyhydroxyheptanoic acid, poly(ε-caprolactone) (PCL), polytrimethylene carbonate, poly 2,2-dimethyltrimethylene carbonate, polydioxanone, polybutyrolactone, polyvalerolactone, polymalic acid, polyethylene adipate, polyethylene succinate, polybutylene adipate, and polybutylene succinate. The polycarbonate is not particularly limited, and examples thereof include tyrosine-polycarbonate.

Alternatively, the first biodegradable polymer and the second biodegradable polymer may be a copolymer obtained by optionally copolymerizing monomers constituting the polymer. Here, the copolymer is not particularly limited. Specific examples thereof include PDLLA-PCL, PLLA-r-PCL, PLLA-b-PCL, PLLA-r-PTMC (TMC = trimethylene carbonate), PLLA-r-PDTC (DCT = 2,2-dimethyltrimethylene carbonate), PLLA-b-PTMC, PLLA-b-PDTC, PLGA (poly(lactide-co-glycolide), polyanhydride, polyorthoester, poly(N-(2-hydroxypropyl)methacrylamide), DLPLA-poly(dl-lactide), LPLA-poly(l-lactide), PGA-polyglycolide, PDO-poly(dioxanone), PGA-TMC-poly(glycolide-co-trimethylene carbonate), PGA-LPLA-poly(l-lactide-co-glycolide), PGA-DLPLA-poly(dl-lactide-co-glycolide), LPLA-DLPLA-poly(l-lactide-co-dl-lactide), and PDO-PGA-TMC-poly(glycolide-co-trimethylene carbonate-co-dioxanone, PAE (Polyanhydride esters)-Salicylate (for example, a polymer in which salicylic acid is bonded to both terminals of polylactide anhydride or polyadipic acid) in which salicylic acid is chemically introduced into a polymer main chain.

Each of the polymer and the copolymer may be used alone, may be used in combination of two or more kinds thereof, or may be used in combination of one or more polymers and one or more copolymers. Each of the polymer and the copolymer may be produced by synthesis or may be a commercially available product. The synthesis method is not particularly limited, and known methods can be applied in the same manner or appropriately modified. For example, polylactic acid (PLA), polyglycolic acid (PGA), or a lactic acid-glycolic acid copolymer (PLGA) can be obtained by selecting one having a required structure from L-lactic acid, D-lactic acid, and glycolic acid, using the selected one as a raw material, and performing dehydrating polycondensation. It can also be obtained by selecting one having a required structure from lactide, which is a cyclic dimer of lactic acid, and glycolide, which is a cyclic dimer of glycolic acid, and performing ring-opening polymerization. Lactide includes L-lactide which is a cyclic dimer of L-lactic acid, D-lactide which is a cyclic dimer of D-lactic acid, meso-lactide obtained by cyclic dimerization of D-lactic acid and L-lactic acid, and DL-lactide which is a racemic mixture of D-lactide and L-lactide. In the present disclosure, any lactide can be used.

Among these, the first biodegradable polymer and the second biodegradable polymer are preferably bulk erosion type polymers. The first biodegradable polymer and the second biodegradable polymer are more preferably selected from the group consisting of a homopolymer of one monomer selected from the group consisting of lactic acid, caprolactone, glycolic acid, dioxanone, butyrolactone, valerolactone, hydroxybutyric acid, and trimethylene carbonate, or a copolymer of two or more monomers.

In the embodiment of the present invention, the decomposition time of the porous structure 20 can be substantially the same as the decomposition time of the drug carrying portion 18 (an embodiment configured as described above is referred to as "fourth embodiment"). With this configuration, the drug carrying portion 18 is also decomposed and disappeared earlier or later than the decomposition and disappear of the porous structure 20. Therefore, in most of the period in which the porous structure 20 is present, the drug is continuously released from the drug carrying portion 18 to the biological tissue with which the porous structure 20 is in contact. Therefore, according to this configuration, it is possible to achieve both suppression and prevention of peripheral emboli and reduction and elimination of the risk of tissue inflammation due to contact with the porous structure 20. Here, "the decomposition time of the porous structure 20 is substantially the same as the decomposition time of the drug carrying portion 18" means that the difference between the decomposition time of the porous structure 20 and the decomposition time of the drug carrying portion 18 is within 24 hours, preferably within 10 hours, more preferably within 5 hours, and particularly preferably within 2 hours. Particularly preferably, the decomposition time of the porous structure 20 is the same as the decomposition time of the drug carrying portion 18 (the above difference is 0 hour).

In the fourth embodiment, the decomposition time of the porous structure 20 and the drug carrying portion 18 is, for example, 2 to 6 months, preferably 3 to 5 months, and preferably 3 to 4 months. With such a decomposition time, an endothelial cell layer can be sufficiently formed on the stent 10 while the porous structure 20 suppresses scattering of a plaque and a thrombus at the time of indwelling the living body indwelling object 100. In the present specification, one month is treated as 30 days.

In the fourth embodiment, the porous structure 20 and the drug carrying portion 18 preferably contain the same biodegradable polymer. This makes it easy to adjust the decomposition time substantially the same. That is, the first biodegradable polymer and the second biodegradable polymer are preferably the same polymer. As specific examples of the first biodegradable polymer and the second biodegradable polymer, those similar to the biodegradable polymers exemplified above may be applied. Among these, the first biodegradable polymer and the second biodegradable polymer are preferably selected from the group consisting of a homopolymer of one monomer selected from the group consisting of lactic acid, caprolactone, glycolic acid, dioxanone, butyrolactone, valerolactone, hydroxybutyric acid, and trimethylene carbonate, or a copolymer of two or more monomers. The first biodegradable polymer and the second biodegradable polymer are more preferably selected from the group consisting of a homopolymer of one monomer selected from the group consisting of lactic acid, caprolactone, glycolic acid, and dioxanone, or a copolymer of two or more monomers.

That is, in a preferred embodiment of the present invention, the first biodegradable polymer and the second biodegradable polymer are the same polymer, and the first biodegradable polymer and the second biodegradable polymer are selected from the group consisting of a homopolymer of one monomer selected from the group consisting of lactic acid, caprolactone, glycolic acid, dioxanone, butyrolactone, valerolactone, hydroxybutyric acid, and trimethylene carbonate, or a copolymer of two or more monomers. In a more preferred embodiment of the present invention, the first biodegradable polymer and the second biodegradable polymer are the same polymer, and the first biodegradable polymer and the second biodegradable polymer are selected from the group consisting of a homopolymer of one monomer selected from the group consisting of lactic acid, caprolactone, glycolic acid, and dioxanone, or a copolymer of two or more monomers.

The weight average molecular weight of the biodegradable polymer used in the living body indwelling object 100 is not particularly limited, and is appropriately selected according to a desired decomposition time (biodegradation rate). Specifically, the weight average molecular weight of the biodegradable polymer is preferably 10,000 or more, more preferably 50,000 to 1,000,000, and still more preferably 100,000 to 500,000. The number average molecular weight of the biodegradable polymer is preferably 5,000 or more, more preferably 20,000 to 800,000, and still more preferably 30,000 to 200,000. Note that examples of a method for measuring the molecular weight (weight average molecular weight, number average molecular weight) include gel permeation chromatography (GPC), a light scattering method, a viscosity measurement method, and a mass spectrometry method (TOFMASS or the like). In the present specification, the weight average molecular weight is a value measured by GPC using polystyrene as a standard substance.

In another embodiment of the present invention, the decomposition time of the porous structure 20 is shorter than the decomposition time of the drug carrying portion 18 (an embodiment configured as described above is referred to as "fifth embodiment"). With this configuration, after the porous structure 20 is decomposed and disappeared to remain only the stent 10, the drug carrying portion 18 is decomposed and disappeared. Therefore, while the porous structure 20 is present, the drug is continuously released from the drug carrying portion 18 to the biological tissue with which the porous structure 20 is in contact. Therefore, according to this configuration, it is possible to achieve both suppression and prevention of peripheral emboli and reduction and elimination of the risk of inflammation of a biological tissue due to contact with the porous structure 20. In Embodiment 2 (see Fig. 6) and Embodiment 3 (see Fig. 7) described above, when a drug other than the anti-inflammatory agent is used, it is possible to more effectively suppress and prevent the inflammatory reaction induced from the porous structure 20, and it is possible to simultaneously perform other required treatments for a long period of time. Here, the difference between the decomposition time of the porous structure 20 and the decomposition time of the drug carrying portion 18 is 2 hours or more, preferably 3 hours or more, and more preferably 1 month or more. Note that the upper limit of the difference between the decomposition time of the porous structure 20 and the decomposition time of the drug carrying portion 18 is not particularly limited, but is, for example, 22 months or less, preferably 12 months or less, and more preferably 5 months or less. When such a time difference is provided, it is possible to achieve both suppression and prevention of peripheral emboli and reduction and elimination of the risk of tissue inflammation due to contact with the porous structure 20 in a more favorable balance.

In the fifth embodiment, the decomposition time of the porous structure 20 is preferably within 5 months, more preferably within 4 months, still more preferably 2 months or more and 4 months or less, and particularly preferably 3 months or more and 4 months or less. The decomposition time of the drug carrying portion 18 is preferably more than 5 months, more preferably more than 6 months, still more preferably more than 7 months and 12 months or less, and particularly preferably more than 8 months and 12 months or less. With such a decomposition time, an endothelial cell layer can be sufficiently formed on the stent 10 while the porous structure 20 suppresses scattering of a plaque and a thrombus at the time of indwelling the living body indwelling object 100.

In the fifth embodiment, the first biodegradable polymer constituting the porous structure 20 and the second biodegradable polymer constituting the drug carrying portion 18 may be a combination of materials in which the decomposition time of the porous structure 20 is shorter than the decomposition time of the drug carrying portion 18. As the first biodegradable polymer and the second biodegradable polymer, those similar to the biodegradable polymers exemplified above may be applied. For example, a biodegradable polymer having a relatively high biodegradation rate, such as polyglycolic acid, a glycolic acid-lactic acid copolymer containing glycolic acid in an amount of more than 10 mol% and less than 100 mol% (preferably 25 mol% or more and 95 mol% or less, more preferably more than 50 mol% and 95 mol% or less) of all monomers (a glycolic acid-L lactic acid copolymer, a glycolic acid-D lactic acid copolymer, a glycolic acid-DL lactic acid copolymer, preferably a glycolic acid-L lactic acid copolymer, a glycolic acid-D lactic acid copolymer), a glycolic acid-caprolactone copolymer containing caprolactone in an amount of 4 mol% or more (preferably 5 mol% or more and 40 mol% or less, more preferably 10 mol% or more and 30 mol% or less) of all monomers, or a caprolactone-lactic acid copolymer containing caprolactone in an amount of more than 44 mol% and 50 mol% or less (preferably 5 mol% or more and 40 mol% or less, more preferably 8 mol% or more and 20 mol% or less) of all monomers (ε-caprolactone-L lactic acid copolymer, ε-caprolactone-D lactic acid copolymer, ε-caprolactone-DL lactic acid copolymer, particularly ε-caprolactone-DL lactic acid copolymer), is preferably used for the porous structure 20. That is, the first biodegradable polymer is preferably at least one selected from the group consisting of polyglycolic acid, polycaprolactone, a glycolic acid-lactic acid copolymer, a caprolactone-lactic acid copolymer, and a glycolic acid-caprolactone copolymer. For example, a biodegradable polymer containing many constituent units derived from lactic acid, such as polylactic acid, a lactic acid-glycolic acid copolymer containing lactic acid in an amount of 90 mol% or more of all monomers, and a lactic acid-ε-caprolactone copolymer containing lactic acid in an amount of 96 mol% or more of all monomers, or polydioxanone is preferably used for the drug carrying portion 18. That is, the second biodegradable polymer is preferably at least one selected from the group consisting of polylactic acid, a lactic acid-ε-caprolactone copolymer, a lactic acid-glycolic acid copolymer, and polydioxanone.

That is, it is preferable that the first biodegradable polymer and the second biodegradable polymer are different polymers, the first biodegradable polymer is at least one selected from the group consisting of polyglycolic acid, polycaprolactone, a glycolic acid-lactic acid copolymer, a caprolactone-lactic acid copolymer, and a glycolic acid-caprolactone copolymer, and the second biodegradable polymer is at least one selected from the group consisting of polylactic acid, a lactic acid-ε-caprolactone copolymer, a lactic acid-glycolic acid copolymer, and polydioxanone.

It is more preferable that the first biodegradable polymer and the second biodegradable polymer are different polymers, the first biodegradable polymer is at least one selected from the group consisting of polyglycolic acid and a glycolic acid-lactic acid copolymer, and the second biodegradable polymer is at least one selected from the group consisting of polylactic acid, a lactic acid-ε-caprolactone copolymer, and polydioxanone.

In addition to the combination of the above materials, the following method can also be used as a method for adjusting the decomposition time of the porous structure 20 to be shorter than the decomposition time of the drug carrying portion 18. Examples of a method for controlling the decomposition time of the porous structure 20 include a method for controlling the composition (for example, glass transition temperature), molecular weight (for example, the length of the yarn constituting the porous structure 20), and crystallinity (for example, spinning conditions of the porous structure 20, presence or absence of annealing) of the first biodegradable polymer (for example, when the crystallinity is small, the decomposition time is shortened, for example, the crystallinity of the porous structure 20 is controlled to 0% or more and 40% or less), a method for appropriately selecting the presence or absence of end treatment (for example, when the end treatment is performed by esterification, the decomposition time becomes longer), and a method using an additive. Among them, a method for controlling the molecular weight, composition (glass transition temperature), or crystallinity of the first biodegradable polymer is preferably used from the viewpoint of ease of controlling the decomposition time. Examples of a method for controlling the decomposition time of the drug carrying portion 18 include a method for controlling the molecular weight, composition, or crystallinity of the second biodegradable polymer (for example, when the crystallinity is high, the decomposition time becomes longer, for example, the crystallinity of the second biodegradable polymer is controlled to be more than 40%), a method for appropriately selecting the presence or absence of end treatment (for example, when the end treatment is performed by esterification, the decomposition time becomes longer), a method for controlling the blending ratio of the drug and the mixing ratio and mixing method of the second biodegradable polymer and the drug, a method using an additive, and a method for controlling the shape of the drug. Among these methods, a method for controlling the molecular weight, composition, or crystallinity of the second biodegradable polymer, and a method for controlling the blending ratio of the drug and the mixing ratio and mixing method of the second biodegradable polymer and the drug are preferably used from the viewpoint of ease of controlling the decomposition time of the drug carrying portion 18 (second biodegradable polymer). Usually, when the molecular weight (weight average molecular weight) of the biodegradable polymer increases, the biodegradation rate decreases. Therefore, a method for adjusting the molecular weight (weight average molecular weight) of the first biodegradable polymer used for the porous structure 20 to be smaller than the molecular weight (weight average molecular weight) of the second biodegradable polymer used for the drug carrying portion 18 can be used.

The molecular weight (weight average molecular weight) of the first biodegradable polymer is, for example, 250,000 or less, 100,000 or more and 200,000 or less, more preferably 120,000 or more and 180,000 or less, and more preferably 140,000 or more and 180,000 or less. The molecular weight (weight average molecular weight) of the second biodegradable polymer is, for example, 50,000 or more, 100,000 or more and 500,000 or less, more preferably 100,000 or more and 250,000 or less, and particularly preferably 100,000 or more and 120,000 or less. The molecular weight (number average molecular weight) of the first biodegradable polymer is, for example, 150,000 or less, and more preferably 30,000 or more and 100,000 or less. The molecular weight (number average molecular weight) of the second biodegradable polymer is, for example, 30,000 or more, and more preferably 40,000 or more and 150,000 or less.

The crystallinity of the first biodegradable polymer is, for example, 0% or more and 40% or less, more preferably 0% or 20% or more and 40% or less, and more preferably 20% or more and 40% or less.

The crystallinity of the second biodegradable polymer is, for example, 0% or more and 50% or less, preferably 0% or more than 40% and 45% or less, and more preferably 0%.

Alternatively, the porous structure 20 may contain a biodegradable polymer previously irradiated with an electron beam. In general, when the (co)polymer is irradiated with an electron beam, a bond in the (co)polymer is easily broken, and the biodegradation rate is increased. Therefore, even when a biodegradable polymer having the same composition is used, the decomposition time can be adjusted by irradiating the biodegradable polymer used for the porous structure 20 with an electron beam in advance.

For example, the decomposition time and the tensile strength maintaining period of polyglycolic acid (crystallinity = 38%, weight average molecular weight = 140,000, number average molecular weight = 70,000, with end treatment by esterification) (polyglycolic acid A) are 1.9 months and 15 days, respectively;
the decomposition time and the tensile strength maintaining period of the glycolic acid-L lactic acid copolymer (glycolic acid : L-lactic acid = 90 : 10 (molar ratio), crystallinity = 23%, weight average molecular weight = 180,000, number average molecular weight = 85,000, with end treatment by esterification) (glycolic acid-L lactic acid copolymer B) are 2.1 months and 15 days, respectively;
the decomposition time and the tensile strength maintaining period of the glycolic acid-caprolactone copolymer (glycolic acid : caprolactone = 75 : 25 (molar ratio), crystallinity = 22%, weight average molecular weight = 120,000, number average molecular weight = 60,000, with end treatment by esterification) (glycolic acid-caprolactone copolymer C) are 3.5 months and 9 days, respectively;
the decomposition time and the tensile strength maintaining period of the caprolactone-DL lactic acid copolymer (caprolactone : DL-lactic acid = 10 : 90 (molar ratio), crystallinity = 0%, weight average molecular weight = 120,000, number average molecular weight = 55,000, with end treatment by esterification) (caprolactone-DL lactic acid copolymer D) are 4 months and 7 days, respectively;
the decomposition time and the tensile strength maintaining period of the glycolic acid-DL lactic acid copolymer (glycolic acid : DL-lactic acid = 25 : 75 (molar ratio), crystallinity = 0%, weight average molecular weight = 120,000, number average molecular weight = 45,000, with end treatment by esterification) (glycolic acid-DL lactic acid copolymer E) are 1 month and 3 days, respectively;
the decomposition time and the tensile strength maintaining period of poly-L lactic acid (crystallinity = 48%, weight average molecular weight = 200,000, number average molecular weight = 120,000, with end treatment by esterification) (poly-L lactic acid F) are 24 months and 7 months, respectively;
the decomposition time and the tensile strength maintaining period of polydioxanone (crystallinity = 45%, weight average molecular weight = 110,000, number average molecular weight = 45,000, with end treatment by esterification) (polydioxanone G) are 8 months and 1.5 months, respectively; and
the decomposition time and the tensile strength maintaining period of poly-D,L lactic acid (crystallinity = 0%, weight average molecular weight = 100,000, number average molecular weight = 50,000) (poly-DL lactic acid H) are 12 months, respectively.

Thus, a combination of the first biodegradable polymer and the second biodegradable polymer may be selected from polyglycolic acid A and poly-L lactic acid F; a glycolic acid-L lactic acid copolymer B and poly-L lactic acid F; a glycolic acid-caprolactone copolymer C and poly-L lactic acid F; a caprolactone-DL lactic acid copolymer D and poly-L lactic acid F; a glycolic acid-DL lactic acid copolymer E and poly-L lactic acid F; polyglycolic acid A and polydioxanone G; a glycolic acid-L lactic acid copolymer B and polydioxanone G; a glycolic acid-caprolactone copolymer C and polydioxanone G; a caprolactone-DL lactic acid copolymer D and polydioxanone G; a glycolic acid-DL lactic acid copolymer E and polydioxanone G; polyglycolic acid A and poly-DL lactic acid H; a glycolic acid-L lactic acid copolymer B and poly-DL lactic acid H; a glycolic acid-ε-caprolactone copolymer C and poly-DL lactic acid H; a ε-caprolactone-DL lactic acid copolymer D and poly-DL lactic acid H; and a glycolic acid-DL lactic acid copolymer E and poly-DL lactic acid H.

In an embodiment of the present invention, a combination of the first biodegradable polymer and the second biodegradable polymer is selected from polyglycolic acid A and poly-L lactic acid F; a glycolic acid-L lactic acid copolymer B and poly-L lactic acid F; a glycolic acid-caprolactone copolymer C and poly-L lactic acid F; a caprolactone-DL lactic acid copolymer D and poly-L lactic acid F; polyglycolic acid A and polydioxanone G; a glycolic acid-L lactic acid copolymer B and polydioxanone G; a glycolic acid-caprolactone copolymer C and polydioxanone G; a caprolactone-DL lactic acid copolymer D and polydioxanone G; polyglycolic acid A and poly-DL lactic acid H; a glycolic acid-L lactic acid copolymer B and poly-DL lactic acid H; a glycolic acid-ε-caprolactone copolymer C and poly-DL lactic acid H; and an ε-caprolactone-DL lactic acid copolymer D and poly-DL lactic acid H.

In an embodiment of the present invention, a combination of the first biodegradable polymer and the second biodegradable polymer is selected from polyglycolic acid A and poly-L lactic acid F; a glycolic acid-L lactic acid copolymer B and poly-L lactic acid F; a glycolic acid-caprolactone copolymer C and poly-L lactic acid F; and a caprolactone-DL lactic acid copolymer D and poly-L lactic acid F. In an embodiment of the present invention, a combination of the first biodegradable polymer and the second biodegradable polymer is selected from polyglycolic acid A and poly-L lactic acid F; and a glycolic acid-L lactic acid copolymer B and poly-L lactic acid F.

In an embodiment of the present invention, a combination of the first biodegradable polymer and the second biodegradable polymer is selected from polyglycolic acid A and polydioxanone G; a glycolic acid-L lactic acid copolymer B and polydioxanone G; a glycolic acid-caprolactone copolymer C and polydioxanone G; and a caprolactone-DL lactic acid copolymer D and polydioxanone G. In an embodiment of the present invention, a combination of the first biodegradable polymer and the second biodegradable polymer is selected from polyglycolic acid A and polydioxanone G; and a glycolic acid-L lactic acid copolymer B and polydioxanone G.

In an embodiment of the present invention, a combination of the first biodegradable polymer and the second biodegradable polymer is selected from polyglycolic acid A and poly-DL lactic acid H; a glycolic acid-L lactic acid copolymer B and poly-DL lactic acid H; a glycolic acid-ε-caprolactone copolymer C and poly-DL lactic acid H; and ε-caprolactone-DL lactic acid copolymer D and poly-DL lactic acid H. In an embodiment of the present invention, a combination of the first biodegradable polymer and the second biodegradable polymer is selected from polyglycolic acid A and poly-DL lactic acid H; and a glycolic acid-L lactic acid copolymer B and poly-DL lactic acid H.

In an embodiment of the present invention, a combination of the first biodegradable polymer and the second biodegradable polymer is selected from a glycolic acid-L lactic acid copolymer B and poly-L lactic acid F; a glycolic acid-caprolactone copolymer C and poly-L lactic acid F; a caprolactone-DL lactic acid copolymer D and poly-L lactic acid F; a glycolic acid-L lactic acid copolymer B and polydioxanone G; a glycolic acid-caprolactone copolymer C and polydioxanone G; a caprolactone-DL lactic acid copolymer D and polydioxanone G; a glycolic acid-L lactic acid copolymer B and poly-DL lactic acid H; a glycolic acid-ε-caprolactone copolymer C and poly-DL lactic acid H; and an ε-caprolactone-DL lactic acid copolymer D and poly-DL lactic acid H. The living body indwelling object 100 in this combination can be suitably used particularly for heart diseases such as acute coronary syndrome (ACS), acute myocardial infarction (AMI), and ST-elevation myocardial infarction (STEMI).

In an embodiment of the present invention, the combination of the first biodegradable polymer and the second biodegradable polymer is polydioxanone G and poly-L lactic acid F. The living body indwelling object 100 in this combination can be suitably used particularly for carotid artery stenosis.

The size of the porous structure 20 is not particularly limited, and is set within a range in which the performance of the stent main body such as the reachability (delivery property) to the lesion and the irritation to the blood vessel wall is not significantly impaired. Preferably, the porous structure 20 is disposed to cover the entire outer peripheral surface of the stent 10. The thickness of the porous structure 20 (the outer diameter of the strand portion) is not particularly limited, and is set within a range that does not significantly impair the performance of the stent 10 such as the delivery property to the lesion and the irritation to the blood vessel wall. Specifically, the thickness of the porous structure 20 (the thickness in the radial direction on the cross-sectional view in cross-sectional view orthogonal to the axial direction illustrated in Figs. 5 to 7) is not particularly limited, and is set within a range that does not significantly impair the performance of the stent 10 such as the reachability (delivery property) to the lesion and the irritation to the blood vessel wall.

The thickness of the porous structure 20 is preferably 1 µm to 100 µm, and more preferably 5 µm to 50 µm. With the above thickness, it is possible to more effectively suppress and prevent peripheral emboli associated with scattering of a plaque and a thrombus at the time of indwelling or after the indwelling of the stent 10. The function related to the thickness of the porous structure 20 is preferably thin as long as the mechanical strength is maintained. A thinner porous structure 20 is advantageous from the viewpoint of obtaining foldability, preventing an increase in the diameter of the device profile, and improving the lesion passage property. That is, when the porous structure 20 is formed to be excessively thick, it is disadvantageous from the viewpoint of foldability and a device profile, and the stent inner diameter (expansion diameter) decreases after being indwelt in the lesion, and the effect of improving the blood flow decreases. As a result, it is considered that thrombus adhesion is also likely to occur. Note that the thickness of the stent 10 in the radial direction (thickness in the radial direction on the cross-sectional view in cross-sectional view orthogonal to the axial direction illustrated in Figs. 5 to 7) can be configured to be larger than the thickness of the porous structure 20. When the thickness of the porous structure 20 is configured to have the dimensions exemplified above, the thickness of the stent 10 can be configured to be, for example, 60 µm to 120 µm.

As a method for manufacturing the porous structure 20, a method for covering the stent 10 with the porous structure 20, and the like, for example, the methods described in WO 2008/062414 A and the like can be similarly applied or appropriately modified and applied.

In the living body indwelling object 100, the drug carrying portion 18 contains a drug in addition to the second biodegradable polymer. Here, the drug contained in the drug carrying portion 18 is not particularly limited, and can be appropriately selected according to a desired use. Considering the induction of inflammation by the porous structure 20, the drug preferably contains an anti-inflammatory agent or an immunosuppressive agent. Here, the anti-inflammatory agent is not particularly limited, and a known anti-inflammatory agent can be used. The anti-inflammatory agent is a steroidal anti-inflammatory agent, a non-steroidal anti-inflammatory agent, or a combination thereof. Examples of the anti-inflammatory agent include alclofenac, alclometasone dipropionate, algestone acetonide, alpha amylase, amcinafal, amcinafide, amfenac sodium, amiprylose hydrochloride, anakinra, anirolac, anitrazafen, apazone, balsalazide disodium, bendazac, benoxaprofen, benzydamine hydrochloride, bromelain, broperamole, budesonide, carprofen, cicloprofen, cintazone, cliprofen, clobetasol propionate, clobetasone butyrate, clopirac, cloticasone propionate, cormethasone acetate, cortodoxon, deflazacort, desonide, desoximetasone, dexamethasone, dexamethasone dipropionate, diclofenac potassium, diclofenac sodium, diflorasone diacetate, diflumidone sodium, diflunisal, diflupredonate, diphthalone, dimethylsulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, prednisolone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarine chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroid, glucocorticoid, tacrolimus, pimecrolimus, prodrugs thereof, and co-drugs thereof. The anti-inflammatory agent may be used singly or in combination of two or more kinds thereof.

The immunosuppressive agent is also not particularly limited, and a known immunosuppressive agent can be used. Examples thereof include sirolimus, everolimus, biolimus A9, pimecrolimus, zotarolimus, a sirolimus derivative such as ABT-578, biolimus (for example, biolimus A9 (registered trademark)), tacrolimus, azathioprine, cyclosporine, cyclophosphamide, mycophenolate mofetil, and gusperimus. The immunosuppressive agent may be used singly or in combination of two or more types thereof. The immunosuppressive agent may be used in combination with an anti-inflammatory agent.

Here, the content of the drug in the drug carrying portion 18 is not particularly limited as long as it is such an amount that a desired drug efficacy is exhibited, but it is preferable to contain a large amount of the drug from the viewpoint of further exhibiting an anti-inflammatory action. Specifically, the content of the drug in the drug carrying portion 18 is preferably 30 mass% or more and 60 mass% or less, and more preferably 45 mass% or more and 50 mass% or less with respect to the total amount (100 mass%) of the second biodegradable polymer and the drug. With this composition, an appropriate amount of the drug can be effectively sustainably released for a predetermined period.

In an embodiment of the present invention, the drug carrying portion 18 is a single layer composed of ε-caprolactone-DL lactic acid copolymer D (ε-caprolactone : DL-lactic acid = 10 : 90 (molar ratio), crystallinity = 0%, weight average molecular weight = 120,000, number average molecular weight = 55,000, decomposition time = 4 months) and sirolimus at a mixing ratio of ε-caprolactone-DL lactic acid copolymer D and sirolimus of 50 : 50 (mass ratio) (additive: none, drug conformation: amorphous state).

In an embodiment of the present invention, the drug carrying portion 18 is a single layer composed of poly-DL lactic acid H (crystallinity = 0%, weight average molecular weight = 100,000, number average molecular weight = 50,000, decomposition time = 12 months) and biolimus A9 (registered trademark) at a mixing ratio of poly-DL lactic acid H and biolimus A9 (registered trademark) of 50 : 50 (mass ratio) (additive: none, drug conformation: amorphous state).

The drug carrying portion 18 may contain other drugs in addition to the anti-inflammatory agent. When the drug carrying portion 18 further contains other drugs, the other drugs are not particularly limited, but a drug that suppresses stenosis and occlusion of the vascular system which may occur when the stent is indwelt in the lesion is preferable. Specific examples thereof include anticancer agents such as paclitaxel, docetaxel, vinblastine, vindesine, irinotecan, and pirarubicin; immunosuppressive agents such as sirolimus, everolimus, pimecrolimus, a sirolimus derivative such as ABT-578, tacrolimus, azathioprine, cyclosporine, cyclophosphamide, mycophenolate mofetil, and gusperimus; antibiotics such as mitomycin, adriamycin, doxorubicin, actinomycin, daunorubicin, idarubicin, pirarubicin, aclarubicin, epirubicin, and zinostatin stimalamer; antithrombotic drugs such as aspirin and ticlopidine; HMG-CoA reductase inhibitors such as cerivastatin, cerivastatin sodium, atorvastatin, pitavastatin, fluvastatin, fluvastatin sodium, simvastatin, and lovastatin; ACE inhibitors such as quinapril, trandolapril, temocapril, delapril, enalapril maleate, and captopril; calcium antagonists such as nifedipine, nilvadipine, benidipine, and nisoldipine; antihyperlipidemic drugs such as probucol; integrin inhibitors such as AJM300; anti-allergic agent such as tranilast; antioxidants such as α-tocopherol, catechin, dibutylhydroxytoluene, and butylhydroxyanisole; GPIIbIIIa antagonists such as abciximab; retinoids such as all-trans retinoic acid; lipid-improving agents such as eicosapentaenoic acid; and antiplatelet drugs such as ticlopidine, cilostazol (500), and clopidogrel. These drugs are preferred in that they can control the behavior of cells in the lesion tissue to treat the lesion. The other drugs may be used singly or in combination of two or more types thereof.

A method of forming the drug carrying portion 18 is not particularly limited, and for example, a method of mixing the second biodegradable polymer and the drug, and at least one of a solvent and another drug as necessary to obtain a mixture, and applying the mixture to a predetermined site of the stent 10 (method for manufacturing the living body indwelling object 100 of the first embodiment and the second embodiment described above), a method of mixing the second biodegradable polymer and the drug, and at least one of a solvent and another drug as necessary to obtain a mixture, and molding the stent 10 using the mixture (method for manufacturing the living body indwelling object 100 of the third embodiment described above), and the like can be used.

As described above, the living body indwelling object 100 according to the present embodiment includes the expandable tubular stent 10 and the porous structure 20 made of a first biodegradable polymer, the porous structure being disposed to cover the stent 10, in which the stent 10 includes the drug carrying portion 18 containing a second biodegradable polymer and a drug, and a decomposition time of the porous structure 20 is substantially the same as a decomposition time of the drug carrying portion 18 or shorter than a decomposition time of the drug carrying portion 18.

According to the living body indwelling object 100 configured as described above, after a certain period of time elapses after indwelling, the porous structure 20 is decomposed and disappeared, and the release of the drug from the stent 10 is continued until the porous structure 20 is decomposed and disappeared. Therefore, the living body indwelling object 100 can effectively suppress the onset of inflammation derived from the porous structure 20 made of a biodegradable polymer while preventing scattering of a plaque and a thrombus at the time of expansion of the stent 10.

The present application is based on Japanese Patent Application No. 2023-169879 filed on September 29, 2023, the disclosure content of which is incorporated herein by reference in its entirety.

### Reference Signs List

- 10: Stent
- 11: Ring
- 12: Link portion
- 18: Drug carrying portion
- 19: Surface of ring
- 19a: Facing portion
- 20: Porous structure
- 100: Living body indwelling object

## Claims

1. A living body indwelling object comprising an expandable tubular stent and a porous structure made of a first biodegradable polymer, the porous structure being disposed to cover the stent, wherein
the stent includes a drug carrying portion containing a second biodegradable polymer and a drug, and
a decomposition time of the porous structure is substantially the same as a decomposition time of the drug carrying portion or shorter than a decomposition time of the drug carrying portion.

2. The living body indwelling object according to claim 1, wherein the stent is made of a non-biodegradable material, and
the drug carrying portion is disposed only in a portion of a surface of a ring forming the stent, the portion facing the porous structure.

3. The living body indwelling object according to claim 1, wherein the stent is made of a non-biodegradable material, and
the drug carrying portion is disposed to cover an entire range of a surface of a ring forming the stent.

4. The living body indwelling object according to claim 1, wherein the entire stent is configured by the drug carrying portion.

5. The living body indwelling object according to any one of claims 1 to 4, wherein the first biodegradable polymer and the second biodegradable polymer are the same polymer, and
the first biodegradable polymer and the second biodegradable polymer are selected from the group consisting of a homopolymer of one monomer selected from the group consisting of lactic acid, caprolactone, glycolic acid, dioxanone, butyrolactone, valerolactone, hydroxybutyric acid, and trimethylene carbonate, or a copolymer of two or more monomers.

6. The living body indwelling object according to any one of claims 1 to 4, wherein the first biodegradable polymer and the second biodegradable polymer are different polymers,
the first biodegradable polymer is at least one selected from the group consisting of polyglycolic acid, polycaprolactone, a glycolic acid-lactic acid copolymer, an ε-caprolactone-lactic acid copolymer, and a glycolic acid-ε-caprolactone copolymer, and
the second biodegradable polymer is at least one selected from the group consisting of polylactic acid, a lactic acid-ε-caprolactone copolymer, a lactic acid-glycolic acid copolymer, and polydioxanone.

7. The living body indwelling object according to claim 6, wherein a molecular weight of the first biodegradable polymer is 120,000 or more and 180,000 or less, and a molecular weight of the second biodegradable polymer is 100,000 or more and 200,000 or less.

8. The living body indwelling object according to claim 6, wherein the first biodegradable polymer is at least one selected from the group consisting of polyglycolic acid and a polyglycolic acid-polylactic acid copolymer, and
the second biodegradable polymer is at least one selected from the group consisting of polylactic acid, a lactic acid-ε-caprolactone copolymer, and polydioxanone.

9. The living body indwelling object according to claim 6, wherein a molecular weight of the first biodegradable polymer is 140,000 or more and 180,000 or less, and a molecular weight of the second biodegradable polymer is 100,000 or more and 120,000 or less.

10. The living body indwelling object according to claim 1, wherein a decomposition time of the porous structure is within 4 months.

11. The living body indwelling object according to claim 1, wherein a decomposition time of the drug carrying portion is more than 4 months.

12. The living body indwelling object according to claim 8, wherein a number average molecular weight in a state where the porous structure and the drug carrying portion are decomposed is 50 to 800.

13. The living body indwelling object according to claim 2 or 3, wherein the non-biodegradable material is a metal material or a polymer material.

14. The living body indwelling object according to claim 1, wherein the drug contains an anti-inflammatory agent.

15. The living body indwelling object according to claim 1, wherein a ratio of the drug in the drug carrying portion is 40 mass% or more and 55 mass% or less with respect to a total amount of the second biodegradable polymer and the drug.
